# EUROPÄISCHE PATENTANMELDUNG

(11) **EP 4 230 617 A1**
(43) Veröffentlichungstag der Anmeldung: **23.08.2023**
(21) Anmeldenummer: 22156919.7
(22) Anmeldetag: 16.02.2022
(51) Int. Cl.: C07D 209/86

(54) **VERFAHREN ZUR HERSTELLUNG VON N-ALKYLCARBAZOLEN**

(71) Anmelder: Heubach Holding Switzerland Ltd, 4132 Muttenz (CH)
(72) Erfinder: Endres, Andreas, 89264 Weißenborn (DE)
(74) Vertreter: Schuck, Alexander

(57) **Zusammenfassung**

Verfahren zur Herstellung von N-Alkylcarbazolen der allgemeinen Formel 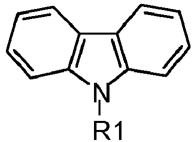 worin R₁ ein linearer oder verzweigter C₁-C₁₂-Alkylrest ist, durch metallfreie Aromatisierung eines teilhydrierten oder perhydrierten N-Alkylcarbazols, wobei das teilhydrierte oder perhydrierte N-Alkylcarbazol in Gegenwart von Aktivkohle unter Inertgasatmosphäre auf eine Temperatur von 160 bis 360°C erhitzt wird.

## Beschreibung

Die Erfindung betrifft ein Verfahren zur Herstellung von N-Alkylcarbazolen der allgemeinen Formel 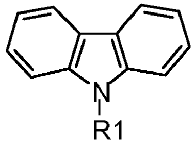 worin R₁ ein linearer oder verzweigter C₁-C₁₂-Alkylrest ist, durch Aromatisierung eines teilhydrierten oder perhydrierten N-Alkylcarbazols.

N-Alkylcarbazole sind wichtige Zwischenprodukte, z.B. für die Herstellung von Pigment Violett 23 (hier ist Alkyl = Ethyl), darüber hinaus werden N-Alkylcarbazole für Anwendungen in der molekularen Elektronik, den Materialwissenschaften und in der Herstellung von Dispersionsfarbstoffen benötigt. In jüngerer Zeit wurden perhydrierte Derivate von N-Alkylcarbazolen als flüssiger organischer Wasserstoffspeicher für elektrolytisch erzeugten Wasserstoff vorgeschlagen, siehe Papp et al., Nachrichten aus der Chemie 62 (2014), S. 965.

N-Alkylcarbazole sind auch durch Aromatisierung der entsprechenden N-Alkyltetrahydrocarbazole herstellbar. Letztere sind leicht mittels einer Fischer-Indolsynthese aus Cyclohexanon und dem entsprechenden N-Alkyl-N-phenylhydrazin herstellbar, wie beschrieben in Fletcher, J. Chem. Soc. (1953), S. 3898. Das N-Ethyl-Die Erfindung betrifft ein Verfahren zur Herstellung von N-Alkylcarbazolen der allgemeinen Formel

Insbesondere das N-Ethylcarbazol ist ein wichtiges Zwischenprodukt, das für die Herstellung des wichtigen Pigments PV 23 benötigt wird. Eine gebräuchliche Methode zur Herstellung von N-Alkylcarbazolen beinhaltet die Alkylierung von aus Steinkohleteer gewonnenem Carbazol mit Alkylierungsmitteln wie Dialkylsulfat, Dialkylcarbonat oder Alkylhalogeniden in Gegenwart von Basen. Nachteilig an dieser Methode ist die schwierige vollständige N-Alkylierung von Carbazol sowie die oft nicht ausreichende Reinheit des erhaltenen Produkts, was auch an den im Carbazol enthaltenen Nebenprodukten, z.B. Acridin, liegt.

N-phenylhydrazin wurde bereits im 19. Jahrhundert synthetisiert, wie beschrieben in E. Fischer, Chem. Ber. 8 (1875), S. 1641.

E. C. Horning et. al., J. Am. Chem. Soc. 70 (1948), S. 3935, beschreiben die Palladiumkatalysierte Herstellung von N-Alkylcarbazolen aus den entsprechenden N-Alkyl-1,2,3,4-tetrahydrocarbazolen. Nachteile dieser Methode sind der hohe Palladiumpreis und der teilweise Verlust des Metalls durch Austrag ("Leaching") sowie die Deaktivierung des Katalysators durch Ablagerung von Nebenprodukten auf dessen Oberfläche.

Die V₂O₅-katalysierte Aromatisierung von nicht am Stickstoff substituierten 1,2,3,4-Tetrahydrocarbazolen, wie beschrieben in Magolan et. al., Synlett 2013. S. 1675, ist auch auf deren N-Alkylderivate anwendbar, nachteilig ist allerdings die sehr lange Reaktionszeit bei erhöhten Temperaturen und die schwierige Entfernung des giftigen Vanadium-Katalysators.

Die seit langem bekannten Aromatisierungen mit 2,3-Dichloro-5,6-dicyano-1,4-benzoquinon (DDQ), Chloranil oder Schwefel sind auch bei N-Alkyl-1,2,3,4-tetrahydrocarbazolen anwendbar, erfordern allerdings die Entfernung der reduzierten p-Chinone bzw. sind mit der Bildung von giftigem Schwefelwasserstoff verbunden. Die lod-Aromatisierung von nicht am Stickstoff substituierten 1,2,3,4-Tetrahydrocarbazolen, wie beschrieben in P. D. Lokande et. al., J. Org. Chem. 80 (2015), S. 2392, benötigt größere Mengen (bis zu 50 mol-%) elementares Iod sowie DMSO als Lösungsmittel bzw. Co-Oxidans, und ist für den industriellen Maßstab weniger geeignet. Zudem kann eine lodierung des Carbazols auftreten. Weiterhin waren die in eigenen Versuchen erzielten Ausbeuten bei der Verwendung von N-Alkyl-1,2,3,4-tetrahydrocarbazolen niedrig.

P. D. Lokande et. al., Tetrahedron Lett. 59 (2018), S. 2145, beschreiben auch die CuCl₂/DMSO-katalysierte Aromatisierung von N-unsubstituierten 1,2,3,4-Tetrahydrocarbazolen. Neben dem toxikologisch bedenklichen Kupfer und dem DMSO waren die in eigenen Versuchen zur Aromatisierung von N-Alkyl-1,2,3,4-tetrahydrocarbazolen erzielten Ausbeuten gering.

Ein recht alter Artikel, Bahr, Chem. Ber. 64 (1931), S. 2258, deutet die prinzipielle Möglichkeit von Aktivkohle katalysierten (Teil)Aromatisierungen an. So beschreibt Bahr die katalytischen Reaktionen des Cyclohexanols an Aktivkohle. Dabei wird vorwiegend durch Wasserabspaltung Cyclohexen gebildet. Durch Dehydrierung bzw. Aromatisierung entstehen daneben Cyclohexanon und Phenol, außerdem wird die Reduktion von Cyclohexen zu Cyclohexan beobachtet.

Somit sind bei der Herstellung von N-Alkylcarbazolen weitere Verbesserungen und Vereinfachungen des Aromatisierungsschrittes wünschenswert. Aufgabe der Erfindung ist es daher, ein einfacheres Verfahren zur Herstellung von N-Alkylcarbazolen umfassend die Aromatisierung von hydrierten N-Alkylcarbazols bereitzustellen.

Gelöst wird die Aufgabe durch ein Verfahren zur Herstellung von N-Alkylcarbazolen der allgemeinen Formel 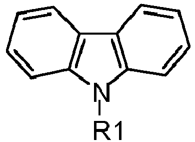 worin R₁ ein linearer oder verzweigter C₁-C₁₂-Alkylrest ist, durch metallfreie Aromatisierung eines teilhydrierten oder perhydrierten N-Alkylcarbazols, wobei das teilhydrierte oder perhydrierte N-Alkylcarbazol in Gegenwart von Aktivkohle unter Inertgasatmosphäre auf eine Temperatur von 160 bis 360°C erhitzt wird, entsprechend dem nachfolgenden Reaktionsschema: 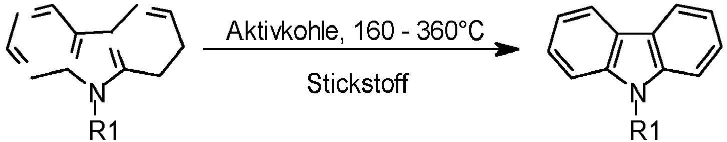 Geeignete teilhydrierte N-Alkylcarbazole sind die isomeren N-Alkyltetrahydrocarbazole, N-Alkylhexahydrocarbazole, N-Alkyloctahydrocarbazole und N-Alkyldecahydrocarbazole. Perhydrierte N-Alkylcarbazole sind N-Alkyldodecahydrocarbazole. Bevorzugte teilhydrierte N-Alkylcarbazole sind N-Alkyl-1,2,3,4,5,6,7,8-octahydrocarbazole, N-Alkyl-1,2,3,4,4a,9a-hexahydrocarbazole und N-Alkyl-1,2,3,4-tetrahydrocarbazole.

Besonders bevorzugte teilhydrierte N-Alkylcarbazole sind N-Alkyl-1,2,3,4-tetrahydrocarbazole.

N-Alkyl-1,2,3,4-tetrahydrocarbazole können durch Fischer-Indolsynthese aus Cyclohexanon und dem entsprechenden N-Alkyl-N-phenylhydrazin hergestellt werden.

R₁ ist bevorzugt ein linearer oder verzweigter C₁-C₈-Alkylrest, insbesondere ist R₁ ein Ethylrest.

Eine speziell bevorzugte Verbindung ist N-Ethyl-1,2,3,4-tetrahydrocarbazol. Diese kann durch Fischer-Indolsynthese aus Cyclohexanon und dem N-Ethyl-N-phenylhydrazin hergestellt werden.

In dem erfindungsgemäßen Verfahren wird die Aromatisierung ohne oder in Gegenwart eines im allgemeinen hochsiedenden organischen Lösungsmittels, z.B. Diethylenglycol, Triethylenglycol, Diglyme, Triglyme, N-Methylpyrrolidon, Ethylencarbonat, Propylencarbonat, Sulfolan, 1,2,4-Trimethylbenzol oder Mesitylen, bevorzugt aber lösemittelfrei durchgeführt. Dazu werden die teilhydrierten oder perhydrierten, bevorzugt die N-Alkyltetrahydrocarbazole, mit Aktivkohle versetzt. Im Allgemeinen ist die Aktivkohle dampfbehandelt und/oder sauer gewaschen. Geeignete Aktivkohlen sind beispielsweise aus Torf hergestellte und anschließend mit Dampf aktivierte und noch sauer gewaschene Aktivkohlen, wie Norit SX2^{®}, sauer hergestellte und nur dampfbehandelte, sowie dampfbehandelte und sauer gewaschene Aktivkohlen aus Torf. Ebenso geeignet sind aus Braunkohle, Steinkohle, Holz, Nussschalen, Tierkohle, landwirtschaftlichen Reststoffen sowie aus Rückständen der Biogasproduktion hergestellte Aktivkohlen mit und ohne Nachbehandlung oder Aktivierung, wie sie für die aus Torf hergestellten Aktivkohlen beschrieben sind. Exemplarisch seien Norit SX2^{®}, Carbopal PA4^{®}, Carbopal MB4^{®} und Carbopal SC11PG^{®} genannt. Die BET-Oberflächen der Aktivkohlen betragen beispielsweise 700 bis 1900m²/g.

Bevorzugt sind aus Torf hergestellte Aktivkohlen.

Die Aktivkohle kann in Mengen von beispielsweise 5 bis 15% (m/m), bezogen auf die Menge des teilhydrierten oder perhydrierten N-Alkylcarbazols, eingesetzt werden.

In einer bevorzugten Ausführungsform der Erfindung wird die Aromatisierung in flüssiger Phase in Abwesenheit eines separaten Lösungsmittels durchgeführt wird.

In weiteren Ausführungsformen der Erfindung wird die Aromatisierung in einem separaten Lösungsmittel durchgeführt. Bevorzugte Lösungsmittel sind ausgewählt ist aus der Gruppe bestehend aus Diethylenglycol, Triethylenglycol, Diglyme, Triglyme, N-Methylpyrrolidon, Ethylencarbonat, Propylencarbonat, Sulfolan, 1,2,4-Trimethylbenzol und Mesitylen.

Die Aromatisierung wird vorzugsweise bei 160 - 320°C, besonders bevorzugt bei 210 - 290°C und insbesondere bei 230 - 270°C durchgeführt. Die Reaktionszeit kann beispielsweise 5 - 25 Stunden, vorzugsweise 5 - 15 Stunden betragen. Im Allgemeinen erfolgt die Umsetzung bei einem Druck von 0,5 bis 5 bar, insbesondere bei Umgebungsdruck.

Die Aromatisierung wird unter Inertgasatmosphäre, im Allgemeinen unter Stickstoffatmosphäre durchgeführt. Zum Abtransport des gebildeten Wasserstoffs wird im Allgemeinen ein leichter Inertgasstrom bzw. Stickstoffstrom über oder durch das Reaktionsgemisch geleitet.

Nach erfolgtem Umsatz (feststellbar beispielsweise durch Kontrolle via DC oder < 5 % (a/a) Edukt im GC bzw. HPLC) kann die Aktivkohle heiß vom Produkt und ggf. dem Lösungsmittel filtriert werden. Das Produkt fällt so bereits sehr sauber an. Überraschenderweise gelingt die Aromatisierung also auch ohne das Vorhandensein von (Edel)metallen. Im Falle einer lösungsmittelfreien Fahrweise kann durch Umkristallisieren des erhaltenen aktivkohlefreien Filtrats, beispielsweise im Falle von N-Ethylcarbazol aus Methanol, ein analysenreines Produkt erhalten werden. Selbstverständlich kann auch die am Ende der Umsetzung erhaltene Reaktionsmischung nach Erkalten aus einem geeigneten Lösungsmittel (im Falle von N-Ethylcarbazol vorzugsweise Methanol) umkristallisiert werden.

Wird die Aromatisierung in Gegenwart eines Lösungsmittels durchgeführt, kann das Rohprodukt durch Zugabe eines geeigneten Lösungsmittels ausgefällt werden und bei Bedarf z.B. durch Umkristallisation oder Destillation weiter aufgereinigt werden.

Eine Vorreinigung der Edukte ist, im Unterschied zur Palladium-katalysierten Aromatisierung, nicht erforderlich. Beim Einsatz von Edukten geringerer Reinheit wird unter Umständen eine größere Menge an Aktivkohle sowie eine längere Reaktionszeit zum vollständigen Umsatz benötigt.

Eine Vorreinigung der N-Alkyl-1,2,3,4-tetrahydrocarbazole kann beispielsweise durch halbstündiges Rühren in etwa 60°C warmem Toluol mit 1-2 % (m/m) Aktivkohle bewerkstelligt werden. Nach Filtration und Entfernen des Lösungsmittels wird reineres N-Alkyl-1,2,3,4-tetrahydrocarbazol erhalten, das mit weniger Aktivkohle schneller als ungereinigtes Startmaterial aromatisiert werden kann. Die Vorreinigung kann z.B. beim N-Ethyl-1,2,3,4-tetrahydrocarbazol auch durch Vakuumdestillation erfolgen.

Die Erfindung wird durch die nachstehenden Beispiele näher erläutert.

### Beispiele

### Beispiel 1

### Herstellung von N-Ethylcarbazol unter Verwendung von Norit SX2^{®}-Aktivkohle

Eine Mischung aus 3,5 g (17,6 mmol) N-Ethyl-1,2,3,4-tetrahydrocarbazol und 500 mg Norit SX2^{®}-Aktivkohle werden unter einem leichten Stickstoffstrom und innigem Rühren fünf Stunden bei einer Außentemperatur von 250°C gerührt. Nach dem Abkühlen der Mischung auf Raumtemperatur wird aus Methanol umkristallisiert. Es werden 2,91 g (14,9 mmol, 85%) N-Ethylcarbazol in Form farbloser Nadeln erhalten.

### Beispiel 2

### Aktivkohleklärung von N-Ethyl-1,2,3,4-tetrahydrocarbazol

Eine Mischung aus 16 g N-Ethyl-1,2,3,4-tetrahydrocarbazol, 45 ml Toluol und 500 mg Norit SX2^{®}-Aktivkohle werden bei 60°C unter Stickstoffatmosphäre 20 Minuten gerührt. Nach dem Abfiltrieren der Aktivkohle wird das Toluol am Rotationsverdampfer entfernt. Es werden 15,7 g (98%) N-Ethyl-1,2,3,4-tetrahydrocarbazol als hellbraunes Öl erhalten, das so für die hier beschriebene Aromatisierung einsetzbar ist.

### Beispiele 3 - 6

### Herstellung von N-Ethylcarbazol unter Verwendung verschiedener Aktivkohlen

Durchführung nach der Arbeitsvorschrift gemäß Beispiel 1, Startmaterial ist immer N-Ethyl-1,2,3,4-tetrahydrocarbazol.

| **Beispiel** | **Aktivkohletyp ^{[1]}** | **Behandlung der Aktivkohle** | **BET-Oberfläche/ [m²/g]** | **Reaktionszeit/ [h] ^{[2]}** | **Ausbeute/ [%]** |
|---|---|---|---|---|---|
| 3 | Carbopal PA4^{®} | Phosphorsäure | 1700 | 15 | 85 |
| 4 | Carbopal MB4^{®} | Dampf | 900 | 15 | 81 |
| 5 | Carbopal SC11PG^{®} | Dampf, saueres Nachwaschen | 950 | 15 | 78 |
| 6 | ohne | - | - | 30 | 2.6 |

| | | | | | |
|---|---|---|---|---|---|
| ^{[1]} Verwendung von 10% (m/m) Aktivkohle ^{[2]} Reaktions-Temperatur 250°C | | | | | |

## Patentansprüche

1. Verfahren zur Herstellung von N-Alkylcarbazolen der allgemeinen Formel 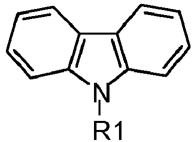 worin R₁ ein linearer oder verzweigter C₁-C₁₂-Alkylrest ist,
durch metallfreie Aromatisierung eines teilhydrierten oder perhydrierten N-Alkylcarbazols, **dadurch gekennzeichnet, dass** das teilhydrierte oder perhydrierte N-Alkylcarbazol in Gegenwart von Aktivkohle unter Inertgasatmosphäre auf eine Temperatur von 160 bis 360°C erhitzt wird.

2. Verfahren nach Anspruch 1, **dadurch gekennzeichnet, dass** ein teilhydriertes N-Alkylcarbazol aromatisiert wird.

3. Verfahren nach Anspruch 2, **dadurch gekennzeichnet, dass** ein N-Alkyl-1,2,3,4-tetrahydrocarbazol der allgemeinen Formel 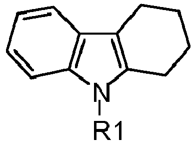 aromatisiert wird.

4. Verfahren nach Anspruch 3, **dadurch gekennzeichnet, dass** das N-Alkyl-1,2,3,4-tetrahydrocarbazol durch Fischer-Indolsynthese aus Cyclohexanon und dem entsprechenden N-Alkyl-N-Phenylhydrazin hergestellt wird.

5. Verfahren nach einem der Ansprüche 1 bis 4, **dadurch gekennzeichnet, dass** R₁ ein linearer oder verzweigter C₁-C₈-Alkylrest ist.

6. Verfahren nach Anspruch 5, **dadurch gekennzeichnet, dass** R₁ Ethyl ist.

7. Verfahren nach einem der Ansprüche 1 bis 6, **dadurch gekennzeichnet, dass** die Aromatisierung in Abwesenheit eines separaten Lösungsmittels durchgeführt wird.

8. Verfahren nach einem der Ansprüche 1 bis 6, **dadurch gekennzeichnet, dass** die Aromatisierung in einem separaten Lösungsmittel durchgeführt wird.

9. Verfahren nach Anspruch 8, **dadurch gekennzeichnet, dass** das Lösungsmittel ausgewählt ist aus der Gruppe bestehend aus Diethylenglycol, Triethylenglycol, Diglyme, Triglyme, N-Methylpyrrolidon, Ethylencarbonat, Propylencarbonat, Sulfolan, 1,2,4-Trimethylbenzol und Mesitylen.

10. Verfahren nach einem der Ansprüche 1 bis 9, **dadurch gekennzeichnet, dass** zur Aromatisierung auf eine Temperatur von 210 bis 290°C erhitzt wird.

11. Verfahren nach einem der Ansprüche 1 bis 10, **dadurch gekennzeichnet, dass** die Aktivkohle aus Torf hergestellt wird.

12. Verfahren nach einem der Ansprüche 1 bis 11, **dadurch gekennzeichnet, dass** die Aktivkohle dampfbehandelt und/oder sauer gewaschen ist.

13. Verfahren nach einem der Ansprüche 1 bis 12, **dadurch gekennzeichnet, dass** die BET-Oberfläche der Aktivkohle 700 bis 1900m²/g beträgt.
